# DEMANDE DE BREVET EUROPEEN

(11) **EP 3 858 243 A1**
(43) Date de publication de la demande: **04.08.2021**
(21) Numéro de dépôt: 21152665.2
(22) Date de dépôt: 21.01.2021
(51) Int. Cl.: A61B 6/00

(54) **SYSTÈME D'IMAGERIE RADIOLOGIQUE COMPRENANT UN DÉTECTEUR PORTABLE D'IMAGE À RAYONS X ET UNE GRILLE ANTI-DIFFUSANTE**

(30) Priorité: 31.01.2020 FR 2000954
(71) Demandeur: Trixell, 38430 Moirans (FR)
(72) Inventeur: BETRAOUI, Farid, 38430 Moirans (FR); GERIN, Nicolas, 38430 Moirans (FR)
(74) Mandataire: Marks & Clerk France

(57) **Abrégé**

Système d'imagerie radiologique (1) comprenant un détecteur portable (2) d'image à rayons X et une grille anti-diffusante (3) comprenant un support (4) de réception du détecteur portable (2), dans lequel :
- le détecteur portable (2) est muni d'un module (5) de communication radio à courte distance ; et
- la grille anti-diffusante (3) comprend une étiquette (6) de communication radio à courte distance, munie d'une mémoire (7) d'au moins 10 kilooctets comprenant des données représentatives de la grille anti-diffusante (3), disposée de manière à être capable de communiquer avec le module (5) de communication radio à courte distance lorsque le détecteur (2) est complètement inséré dans le support (4) de réception ;
le détecteur portable étant configuré pour déterminer localement si la grille anti-diffusante (3) et le détecteur portable (2) sont compatibles ou incompatibles à partir des données représentatives de la grille anti-diffusante (3) lues fournies au module (5) de communication radio à courte distance par l'étiquette (6).

## Description

L'invention porte sur un détecteur portable d'image à rayons X capable de détecter et identifier automatiquement et de manière autonome une grille anti-diffusante. La grille anti-diffusante est un outil utilisé en radiographie pour améliorer la qualité de l'image radiologique, en diminuant la contribution du rayonnement diffusé; cette grille se place entre le patient et le détecteur.

Il est connu le document CN103961116B (Siemens) qui divulgue un système d'identification de grille anti-diffusante comprenant un dispositif d'identification de type code-barres, un lecteur de code barre, et un centre de contrôle distant relié en réseau. Le lecteur de code-barres est utilisé pour identifier et transmettre l'identification de la grille au centre de contrôle distant, ce centre de contrôle associe les informations de la grille anti-diffusante avec des informations relatives au patient pour vérifier si l'association est correcte ou pas et pour enregistrer des informations relatives au générateur à haute tension et au tube à rayons X.

Un tel dispositif est complexe, coûteux, et a une certaine latence car il doit faire appel à un centre de contrôle distant. Il allonge la séquence d'opérations réalisées par l'opérateur radio en lui demandant d'exécuter la lecture du code barre de la grille anti-diffusante avant l'acquisition de l'image ; En effet, pour que la grille anti-diffusante fonctionne, elle doit être en contact avec le détecteur alors que pour que la lecture du code barre se fasse une distance de plusieurs centimètres est nécessaire. Il n'y a pas de dispositif intégré d'alerte de l'utilisateur en cas d'inadéquation entre la grille anti-diffusante et le détecteur.

Il est également connu le document US8634517 (Philips) qui décrit un détecteur radiographique portable avec une grille anti-diffusante capable d'associer le détecteur et le système radiographique pour déterminer les réglages en vue d'une exposition automatique par un détecteur portable à partir d'informations échangées avec le détecteur. Il mentionne l'utilisation de la technologie RFID pour établir la communication entre le support de grille et le détecteur de rayons X. Le détecteur est passif et toute l'information est gérée par un centre de contrôle distant externe à l'ensemble détecteur-grille.

Un tel dispositif est complexe, coûteux, et a une certaine latence car il doit faire appel à un centre de contrôle distant. D'autre part, il n'est pas sécurisé car la communication entre la grille anti-diffusante et le centre de contrôle ne se fait pas en champ proche (elle peut être interceptée). Il n'y a pas de dispositif intégré d'alerte de l'utilisateur en cas d'inadéquation entre la grille anti-diffusante et le détecteur.

Il est également connu d'utiliser un système de capteurs à effet Hall et des aimants pour identifier le type de grille anti-diffusante utilisée. Dans un tel système, le détecteur est équipé de deux capteurs à effet Hall, et des aimants sont introduits dans le support de grille en face des capteurs à effet Hall. Si les capteurs à effet Hall ne détectent aucun aimant, le détecteur considère qu'il n'y a pas de grille anti-diffusante. Le détecteur peut identifier jusqu'à trois types de grilles, grâce aux deux capteurs, selon que l'un d'entre eux ou les deux détectent un aimant. Cette information type de grille est enregistrée dans le détecteur et dans l'image sous la forme d'un code : 0, 1, 2, 3: 0 signifiant "pas de grille", et 1, 2, ou 3 signifiant respectivement grille de type 1, de type 2 ou de type 3.

Un tel dispositif est limité car il ne peut identifier que trois types de grilles. Il n'y a pas de dispositif intégré d'alerte de l'utilisateur en cas d'inadéquation entre la grille anti-diffusante et le détecteur.

Un but de l'invention est de pallier les problèmes précédemment cités, et plus particulièrement de pouvoir de manière autonome, sécurisée, simplifiée et rapide (en local) détecter un nombre quasi-illimité de grilles anti-diffusantes et déterminer si la grille à utiliser est compatible avec le détecteur.

Il est proposé, selon un aspect de l'invention, un système d'imagerie radiologique comprenant un détecteur portable d'image à rayons X et une grille anti-diffusante comprenant un support de réception du détecteur portable, dans lequel :
- le détecteur portable est muni d'un module de communication radio à courte distance ; et
- la grille anti-diffusante comprend une étiquette de communication radio à courte distance, munie d'une mémoire d'au moins 10 kilooctets comprenant des données représentatives de la grille anti-diffusante, disposée de manière à être capable de communiquer avec le module de communication radio à courte distance lorsque le détecteur est complètement inséré dans le support de réception ;
le détecteur portable étant configuré pour déterminer localement (i.e. sans accès distant) si la grille anti-diffusante et le détecteur portable sont compatibles ou incompatibles à partir des données représentatives de la grille anti-diffusante lues fournies au module de communication radio à courte distance par l'étiquette.

Un tel système permet de détecter, de manière autonome, sécurisée, simplifiée et rapide (en local), un nombre quasi-illimité de grilles anti-diffusantes et déterminer si la grille à utiliser est compatible avec le détecteur.

L'identification automatique de la grille anti-diffusante, la vérification de sa compatibilité avec le détecteur et le dispositif intégré d'alerte de l'utilisateur permettent d'éviter une erreur de manipulation susceptible d'exposer inutilement le patient aux rayons X. La capacité du détecteur à réaliser la détection de la grille anti-diffusante de manière autonome permet l'utilisation de ce type de solution sur des équipements de radiologie qui ne sont pas équipés d'un centre de contrôle.

Dans un mode de réalisation, le système comprend un module de blocage du fonctionnement du détecteur portable en le déclarant comme non-prêt à acquérir une image si le détecteur portable et la grille anti-diffusante sont incompatibles.

Ainsi, lorsque la grille anti-diffusante n'est pas compatible avec le détecteur inséré dans son support, le système d'imagerie radiologique est bloqué.

Selon un mode de réalisation, le système comprend un module d'alerte configuré pour signaler à un utilisateur si la grille anti-diffusante et le détecteur portable sont compatibles ou incompatibles.

Ainsi, l'utilisateur est clairement averti de la compatibilité ou de l'incompatibilité du détecteur portable et le la grille anti-diffusante.

Dans un mode de réalisation, le module d'alerte comprend :
- un émetteur sonore configuré pour émettre un premier signal sonore en cas de compatibilité de la grille anti-diffusante et du détecteur portable, et pour émettre un deuxième signal sonore en cas d'incompatibilité de la grille anti-diffusante et du détecteur portable ; et/ou
- un émetteur lumineux configuré pour émettre un premier signal lumineux en cas de compatibilité de la grille anti-diffusante et du détecteur portable, et pour émettre un deuxième signal lumineux en cas d'incompatibilité de la grille anti-diffusante et du détecteur portable.

Selon un mode de réalisation, l'émetteur lumineux comprend des diodes électroluminescentes configurées pour clignoter en vert en cas de compatibilité de la grille anti-diffusante et du détecteur portable, et pour clignoter en rouge en cas d'incompatibilité de la grille anti-diffusante et du détecteur portable.

L'utilisation d'un code visuel si classique est aisément compréhensible immédiatement.

Selon un mode de réalisation, le détecteur portable est configuré pour, lors d'une acquisition d'image, appliquer des corrections d'images correspondant au type de la grille anti-diffusante.

Ainsi, le traitement de l'image acquise est amélioré.

Par exemple, le détecteur portable est configuré pour, lors d'une acquisition d'image, insérer, dans l'en-tête du fichier informatique représentant l'image acquise, les données représentatives de la grille anti-diffusante.

Ainsi, de manière rapide, locale et efficace, les caractéristiques de la grille anti-diffusante utilisée pour acquérir l'image est dans le fichier informatique représentant l'image acquise, et le système peut alors utiliser ces caractéristiques comme paramètres d'entrée du traitement d'image.

Dans un mode de réalisation, les données représentatives de la grille anti-diffusante comprennent un identifiant représentatif du type de grille anti-diffusante, et/ou un identifiant représentatif de la grille anti-diffusante, et/ou un identifiant représentatif de son orientation portrait ou paysage, et/ou un identifiant représentatif du nombre de paires de lignes par centimètres, et/ou un identifiant représentatif du facteur de forme, et/ou un identifiant représentatif de la distance focale nominale de la source à rayons X, et/ou un identifiant représentatif des distances focales minimum et maximum de la source à rayons X, et/ou un identifiant représentatif du taux de transmission primaire des rayons X du dispositif, et/ou un identifiant représentatif des dimensions de la grille anti-diffusante, et/ou un identifiant représentatif de l'épaisseur du matériau absorbant.

Toutes ces données permettent d'identifier précisément et localement le type de grille et ses caractéristiques.

Par exemple, le module de communication radio à courte distance est de type communication en champ proche NFC pour acronyme de "Near Field Communication" en langue anglaise, et l'étiquette de communication radio à courte distance est une étiquette de type NFC.

L'utilisation d'une communication NFC est facile à mettre en œuvre et peu onéreuse car de nombreux formats d'étiquettes ou "tags" en langue anglaise i permettant l'identification sont disponibles, et sont en général peu onéreux. D'autre part, la communication NFC est sécurisée car elle se fait en champ proche.

En variante, le module de communication radio à courte distance est de type communication par radio-identification RFID, et l'étiquette de communication radio à courte distance est une étiquette de type RFID. Celle-ci présente l'inconvénient de ne pas être sécurisée. L'invention sera mieux comprise à l'étude de quelques modes de réalisation décrits à titre d'exemples nullement limitatifs et illustrés par les dessins annexés sur lesquels les figures :
[Fig.1] illustre schématiquement un système d'imagerie radiologique numérique, selon un aspect de l'invention ; et
[Fig.2] illustre schématiquement un détecteur portable d'image à rayons X d'un système de la figure 1, selon un aspect de l'invention.

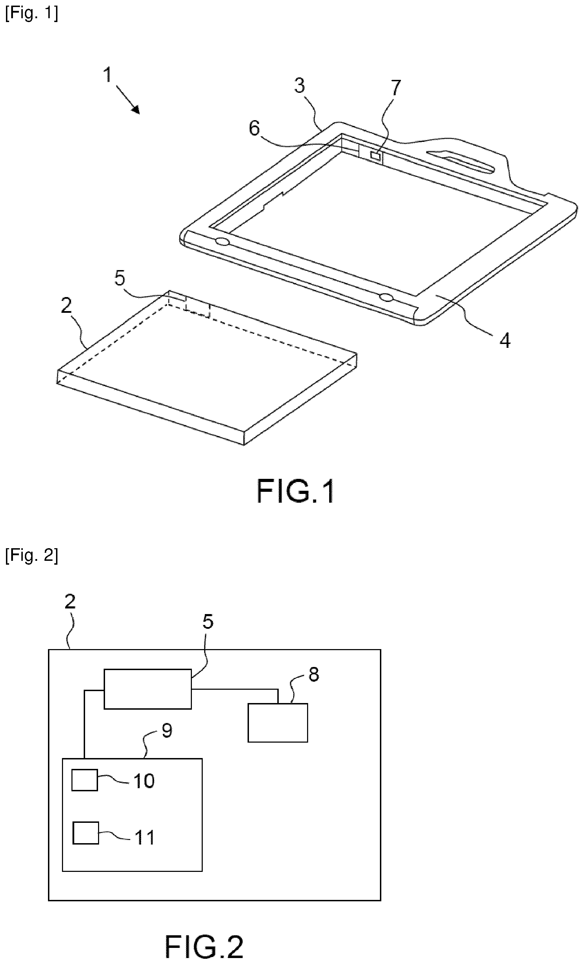

La figure 1 représente schématiquement un système 1 d'imagerie radiologique selon un aspect de l'invention.

Un système d'imagerie radiologique 1 comprend un détecteur portable 2 d'image à rayons X et une grille anti-diffusante 3 comprenant un support 4 de réception du détecteur portable 2, dans lequel :
- le détecteur portable 2 est muni d'un module 5 de communication radio à courte distance ; et
- la grille anti-diffusante 3 comprend une étiquette 6 ou "tag" en langue anglaise de communication radio à courte distance, munie d'une mémoire 7 d'au moins 10 kilooctets comprenant des données représentatives de la grille anti-diffusante 3, disposée de manière à être capable de communiquer avec le module 5 de communication radio à courte distance lorsque le détecteur 2 est complètement inséré dans le support 4 de réception.

Le détecteur portable 2 est configuré pour déterminer localement (sans connexion à un serveur distant) si la grille anti-diffusante 3 et le détecteur portable 2 sont compatibles ou incompatibles à partir des données représentatives de la grille anti-diffusante 3 lues fournies au module 5 de communication radio à courte distance par l'étiquette 6.

La figure 2 représente schématiquement un détecteur portable 2 selon un aspect de l'invention, muni d'un module 8 de blocage du fonctionnement du détecteur portable 2 en le déclarant comme non-prêt à acquérir une image si le détecteur portable (2) et la grille anti-diffusante (3) sont incompatibles.

Ainsi il n'y a pas d'émission de rayons X lorsque le détecteur portable 2 et la grille anti-diffusante 3 sont incompatibles.

Un module 9 d'alerte peut être configuré pour signaler à un utilisateur si la grille anti-diffusante 3 et le détecteur portable 2 sont compatibles ou incompatibles.

Le module 9 d'alerte peut comprendre :
- un émetteur sonore 10 configuré pour émettre un premier signal sonore en cas de compatibilité de la grille anti-diffusante 3 et du détecteur portable 2, et pour émettre un deuxième signal sonore en cas d'incompatibilité de la grille anti-diffusante 3 et du détecteur portable 2 ; et/ou
- un émetteur lumineux 11 configuré pour émettre un premier signal lumineux en cas de compatibilité de la grille anti-diffusante 3 et du détecteur portable 2, et pour émettre un deuxième signal lumineux en cas d'incompatibilité de la grille anti-diffusante 3 et du détecteur portable 2.

Par exemple, l'émetteur lumineux peut comprendre des diodes électroluminescentes configurées pour clignoter en vert en cas de compatibilité de la grille anti-diffusante 3 et du détecteur portable 2, et pour clignoter en rouge en cas d'incompatibilité de la grille anti-diffusante 3 et du détecteur portable 2.

Lorsque le détecteur 2 est inséré entièrement dans le support 4 de la grille anti-diffusante 3, le module 5 de communication radio à courte distance se retrouve face de l'étiquette 6. Si la grille anti-diffusante 3 utilisée est compatible avec le détecteur portable 2, alors celui-ci peut émettre un premier son particulier correspondant à une compatibilité et les diodes électroluminescentes peuvent clignoter en vert.

A contrario, si la grille anti-diffusante 3 n'est pas compatible avec le détecteur portable 2, celui-ci peut émettre un deuxième son correspondant à une incompatibilité et les diodes électroluminescentes peuvent clignoter en rouge.

Le module 5 de communication radio à courte distance lit alors les données ou informations contenues dans la mémoire 7 de l'étiquette, c'est-à-dire toutes les caractéristiques de la grille anti-diffusante 3 utilisée.

Le système 1 d'imagerie radiologique à rayons X peut alors récupérer les informations de la grille anti-diffusante 3 par commande logicielle adressée au détecteur à rayons X et ainsi ajuster les caractéristiques du faisceau à rayons X qu'il va générer pour exposer le patient.

Le détecteur portable 2 peut être configuré pour, lors d'une acquisition d'image, insérer, dans l'en-tête du fichier informatique représentant l'image acquise, les données représentatives de la grille anti-diffusante 3.

Le détecteur 2 à rayons X écrit alors l'ensemble des caractéristiques de la grille anti-diffusante 3 utilisée pour acquérir une image à rayons X dans l'en-tête de l'image numérique (fichier informatique représentant l'image acquise) qu'il a réalisée. Le système utilise alors ces caractéristiques comme paramètres d'entrée pour son traitement d'images.

Ceci permet de réduire les risques d'erreurs des données de la grille anti-diffusante utilisée pour l'acquisition et le traitement de l'image.

Les données représentatives de la grille anti-diffusante 3 peuvent comprendre un identifiant représentatif du type de grille anti-diffusante, et/ou un identifiant représentatif de la grille anti-diffusante, et/ou un identifiant représentatif de son orientation portrait ou paysage, et/ou un identifiant représentatif du nombre de paires de lignes par centimètres, et/ou un identifiant représentatif du facteur de forme, et/ou un identifiant représentatif de la distance focale nominale de la source à rayons X, et/ou un identifiant représentatif des distances focales minimum et maximum de la source à rayons X, et/ou un identifiant représentatif du taux de transmission primaire des rayons X du dispositif, et/ou un identifiant représentatif des dimensions de la grille anti-diffusante, et/ou un identifiant représentatif de l'épaisseur du matériau absorbant.

Le module 5 de communication radio à courte distance peut être de type communication en champ proche NFC et l'étiquette 6 de communication radio à courte distance est une étiquette de type NFC.

En variante, le module 5 de communication radio à courte distance peut être de type communication par radio-identification RFID, et l'étiquette 6 de communication radio à courte distance est une étiquette de type RFID.

## Revendications

1. Système d'imagerie radiologique (1) comprenant un détecteur portable (2) d'image à rayons X et une grille anti-diffusante (3) comprenant un support (4) de réception du détecteur portable (2), dans lequel :
- le détecteur portable (2) est muni d'un module (5) de communication radio à courte distance ; et
- la grille anti-diffusante (3) comprend une étiquette (6) de communication radio à courte distance, munie d'une mémoire (7) d'au moins 10 kilooctets comprenant des données représentatives de la grille anti-diffusante (3), disposée de manière à être capable de communiquer avec le module (5) de communication radio à courte distance lorsque le détecteur (2) est complètement inséré dans le support (4) de réception ;
le détecteur portable étant configuré pour déterminer localement si la grille anti-diffusante (3) et le détecteur portable (2) sont compatibles ou incompatibles à partir des données représentatives de la grille anti-diffusante (3) lues fournies au module (5) de communication radio à courte distance par l'étiquette (6).

2. Système selon la revendication 1, comprenant un module (8) de blocage du fonctionnement du détecteur portable (2) en le déclarant comme non-prêt à acquérir une image si le détecteur portable (2) et la grille anti-diffusante (3) sont incompatibles.

3. Système selon l'une des revendications précédentes, comprenant un module (9) d'alerte configuré pour signaler à un utilisateur si la grille anti-diffusante (3) et le détecteur portable (2) sont compatibles ou incompatibles.

4. Système selon la revendication 3, dans lequel le module (9) d'alerte comprend :
- un émetteur sonore (10) configuré pour émettre un premier signal sonore en cas de compatibilité de la grille anti-diffusante (3) et du détecteur portable (2), et pour émettre un deuxième signal sonore en cas d'incompatibilité de la grille anti-diffusante (3) et du détecteur portable (2) ; et/ou
- un émetteur lumineux (11) configuré pour émettre un premier signal lumineux en cas de compatibilité de la grille anti-diffusante (3) et du détecteur portable (2), et pour émettre un deuxième signal lumineux en cas d'incompatibilité de la grille anti-diffusante (3) et du détecteur portable (2).

5. Système selon la revendication 4, dans lequel l'émetteur lumineux comprend des diodes électroluminescentes configurées pour clignoter en vert en cas de compatibilité de la grille anti-diffusante (3) et du détecteur portable (2), et pour clignoter en rouge en cas d'incompatibilité de la grille anti-diffusante (3) et du détecteur portable (2).

6. Système selon l'une des revendications précédentes, dans lequel le détecteur portable (2) est configuré pour, lors d'une acquisition d'image, appliquer des corrections d'images correspondant au type de la grille anti-diffusante (3).

7. Système selon la revendication 6, dans lequel le détecteur portable (2) est configuré pour, lors d'une acquisition d'image, insérer, dans l'en-tête du fichier informatique représentant l'image acquise, les données représentatives de la grille anti-diffusante (3).

8. Système selon l'une des revendications précédentes, dans lequel les données représentatives de la grille anti-diffusante (3) comprennent un identifiant représentatif du type de grille anti-diffusante, et/ou un identifiant représentatif de la grille anti-diffusante, et/ou un identifiant représentatif de son orientation portrait ou paysage, et/ou un identifiant représentatif du nombre de paires de lignes par centimètres, et/ou un identifiant représentatif du facteur de forme, et/ou un identifiant représentatif de la distance focale nominale de la source à rayons X, et/ou un identifiant représentatif des distances focales minimum et maximum de la source à rayons X, et/ou un identifiant représentatif du taux de transmission primaire des rayons X du dispositif, et/ou un identifiant représentatif des dimensions de la grille anti-diffusante, et/ou un identifiant représentatif de l'épaisseur du matériau absorbant.

9. Système selon l'une des revendications 1 à 8, dans lequel le module (5) de communication radio à courte distance est de type communication en champ proche NFC, et l'étiquette (6) de communication radio à courte distance est une étiquette de type NFC.

10. Système selon l'une des revendications 1 à 8, dans lequel le module (5) de communication radio à courte distance est de type communication par radio-identification RFID, et l'étiquette (6) de communication radio à courte distance est une étiquette de type RFID.
